# EUROPEAN PATENT SPECIFICATION

(11) **EP 3 009 007 B1**
(45) Date of publication and mention of the grant of the patent: **25.10.2017**
(21) Application number: 15196305.5
(22) Date of filing: 25.11.2015
(51) Int. Cl.: A23L 3/42, A01G 33/00, A61K 35/74, A61K 35/748, C12N 1/12

(54) **PROCESS FOR TREATING AN AQUEOUS COMPOSITTION OF ALGAE**
VERFAHREN ZUR BEHANDLUNG EINER WÄSSRIGEN ALGENZUSAMMENSETZUNG
PROCÉDÉ DE TRAITEMENT D'UNE COMPOSITION AQUEUSE D'ALGUES

(30) Priority: 26.11.2014 EP 14194968
(43) Date of publication of application: 20.04.2016
(73) Proprietor: Solvay SA, 1120 Bruxelles (BE)
(72) Inventor: PASCAL, Jean-Philippe, 54600 Villers Les Nancy (FR); AMOUROUX, Magali, 78220 Viroflay (FR); PATAT, Olivier, 75017 Paris (FR)
(74) Representative: Teipel, Stephan

(56) References cited:
- WO-A1-98/25471
- CN-A- 102 726 771
- JP-A- 2004 331 629
- DATABASE WPI Week 200540 Thomson Scientific, London, GB; AN 2005-395916 XP002728964, & WO 2005/048741 A1 (LIZOGUB V A) 2 June 2005 (2005-06-02)
- KIM CHOONG-JAE ET AL: "Optimization of outdoor cultivation of Spirulina platensis and control of contaminant organisms", ALGAE, vol. 21, no. 1, March 2006 (2006-03), pages 133-139, XP002728965, ISSN: 1226-2617
- DATABASE BIOSIS [Online] BIOSCIENCES INFORMATION SERVICE, PHILADELPHIA, PA, US; 1983, VONSHAK A ET AL: "PRODUCTION OF SPIRULINA-PLATENSIS BIO MASS MAINTENANCE OF MONO ALGAL CULTURE OUTDOORS", XP002728966, Database accession no. PREV198376040929
- ORDONEZ-VALENCIA CLAUDIA ET AL: "In vitro antifungal effects of potassium bicarbonate on Trichoderma sp. and Sclerotinia sclerotiorum", MYCOSCIENCE, SPRINGER-VERLAG, TO, vol. 50, no. 5, 17 September 2009 (2009-09-17), pages 380-387, XP019725578, ISSN: 1618-2545, DOI: 10.1007/S10267-009-0495-Z

## Description

The present invention relates to a process for drying an aqueous composition of algae, and more specifically a fresh algal biomass.

The world market for algae is experiencing a great expansion following a considerable demand for products derived from renewable raw materials in fields as varied as human food (colloids such as agars, carrageenans, alginates, proteins, polysaccharides, etc), the cosmetics industry, and the production of biofuels and of biomaterials. The algal biomass is a renewable raw material which has the advantages of not affecting food resources, water consumption, and cultivatable soil quality.

However, in the majority of applications, the algal biomass must be stored before use and this biomass undergoes degradation by fermentation which makes it unfit or less advantageous in the subsequent applications. Improvements in this field are therefore necessary. WO2005048741 discloses a method of treatment and drying of aqueous brown alga, comprising the addition of sodium bicarbonates, with 1.5-2.2% wt, where the aqueous algae composition comprising 5-15% wt water in the composition. The present invention therefore relates, in a first embodiment, to a process for drying an aqueous composition of algae, comprising one or more water removal steps, and according to which at least one bicarbonate is added to at least one of the water removal steps in a total amount, relative to the amount of dry matter of the aqueous composition of algae before addition of the bicarbonate, of at least 0.5% by weight and of at most 8% by weight, and according to which the bicarbonate-supplemented aqueous composition of algae is treated with a stream of air having a flow rate greater than or equal to 20 Nm³/h/kg of composition before treatment with the stream of air and less than or equal to 100 Nm³/h/kg, at a temperature greater than or equal to 25°C and less than or equal to 100°C and for a period greater than or equal to 1 h and less than or equal to 10 h.

One of the essential characteristics of the invention lies in the nature of the additive, the bicarbonate, added to the aqueous composition of algae. Surprisingly and without wishing to be bound by any theoretical explanation, it is thought that the bacteriostatic properties of the bicarbonate slow down the fermentation phenomenon of the composition, due to the action of anaerobic bacteria on the proteins and carbohydrates of the alga present in the composition.

Another essential characteristic of the invention lies in the amount of alkali metal bicarbonate added. Surprisingly and without wishing to be bound by any theoretical explanation, it is thought that there is a bicarbonate content threshold below which no slowing down effect is observed and an upper limit above which the bicarbonate added causes an effect opposite to that desired, probably following an increase in the interstitial ionic strength of the biomass in the aqueous composition of algae, which causes the algal cells to break up.

The advantages linked to the present invention are the following:
- storage life of the supplemented aqueous composition of algae more than 1 day longer compared with a nontreated composition;
- greater time flexibility in the rest of the operations for treating the composition downstream of the storage;
- control and reduction of phenomena of degradation of the algal composition and of phenomena of nauseating and potentially toxic gas release;
- improvements in the organoleptic qualities of the composition treated with alkali metal bicarbonate, whether under severe drying conditions (high-temperature and short drying times) or mild drying conditions (low temperature and long drying times);
- protection against fungal contaminations present in the domestic or working environment (Penicillium brevicompactum,Cladosporium sphaerospermum, Aspergillus niger, ochraceus, flavus, fumigatus, Fusarium graminearum);
- reinforcement of the effectiveness of the other expensive means of storage, such as cold storage (between 1 and 4°C);
- use of an additive perfectly in line with the final uses of the algae, in green chemistry, in the cosmetics industry, in human food and animal feed.

In a second embodiment, the present invention relates to a process for drying an aqueous composition of algae, comprising one or more water removal steps, and according to which at least one bicarbonate is added to at least one of the water removal steps in a total amount, relative to the amount of dry matter of the aqueous composition of algae before addition of the bicarbonate, of at least 0.5% by weight and of at most 8% by weight, and according to which the bicarbonate-supplemented aqueous composition of algae is treated with a stream of air having a flow rate greater than or equal to 800 Nm³/h/kg of composition before treatment with the stream of air and less than or equal to 2000 Nm³/h/kg, at a temperature greater than or equal to 175°C and less than or equal to 220°C and for a period greater than or equal to 1 s and less than or equal to 30 s.

In both embodiments of the invention, the aqueous composition of algae before addition of the bicarbonate generally contains water in a total amount of at least 60% by weight of the composition, preferably of at least 65% by weight and more preferably of at least 70% by weight. This composition generally contains water in a total amount of at most 90% by weight of the composition, preferably of at most 85% by weight and more preferably of at most 80% by weight. An aqueous composition of algae before addition of the bicarbonate containing water in a total amount of approximately 75% by weight of the composition is particularly suitable.

The total amount of water present in the aqueous composition of algae comprises the water which is intracellular with respect to the alga and the water which is extracellular with respect to the alga.

The aqueous composition of algae before addition of the bicarbonate generally contains an amount of dry matter of at least 10% by weight of the composition, preferably of at least 15% by weight and more preferably of at least 20% by weight. This composition generally contains an amount of dry matter of at most 40% by weight of the composition, preferably of at most 35% by weight and more preferably of at most 30% by weight. An aqueous composition of algae before addition of the bicarbonate containing an amount of dry matter of approximately 25% by weight of the composition is particularly suitable. The amount of dry matter is obtained by stoving at 104°C in an aerated thermostatic oven until the weight is constant.

In both embodiments of the invention, the aqueous composition of algae can originate from a culture of algae in an open-air pond or in a bioreactor. The aqueous composition of algae preferably originates from the harvesting of a culture of algae in an open-air pond. The harvesting generally comprises a step of filtering the culture of algae, followed by a step of pressing and/or draining the filtered culture. The aqueous composition of algae that is treated by addition of bicarbonate is preferably an aqueous composition of algae that is obtained after filtering and pressing and/or draining a culture of algae, preferably in an open-air pond. Such a composition will also be identified as a fresh algal biomass.

In both embodiments of the invention, the alga present in the aqueous composition of algae may be of any type. The alga may be a microalga or a macroalga, and is preferably a microalga. The alga is preferably an alga rich in nutrients, such as, for example, carbohydrates, lipids, proteins, vitamins and mineral salts.

In both embodiments of the invention, the alga is quite particularly preferably a spirulina microalga, as defined in "La Spiruline peut-elle etre un atout pour la santé et le développement en Afrique?" ["Can Spirulina be an asset for health and development in Africa?"] August 2008, Loïc Charpy, Marie José Langlade and Romain Alliod, Institut de Recherche pour le Développement [Research Institute for Development] UR 167 (CYROCO) COM, rue de la Batterie des Lions 13007 Marseilles, pages 6 to 8. Spirulina are filamentous cyanobacteria which belong to the *Arthrospira* or *Spirulina* genus depending on the authors. They grow naturally in alkaline water. The group of cyanobacteria, formerly called blue algae and then Cyanophyceae, consists of bacteria capable of photosynthesis with oxygen production. The aqueous composition of algae that is treated with bicarbonate is preferably a fresh algal biomass of spirulina.

Without wishing to be bound by any theoretical explanation, it is thought that algae rich in nutrients are a target of choice for attacks by microorganisms such as fungi and bacteria and also for fermentation phenomena, and that the treatment according to both embodiments of the invention is particularly suitable for this type of algal biomass.

In both embodiments of the invention, the bicarbonate is preferably chosen from alkali metal bicarbonates and ammonium bicarbonate. The alkali metal bicarbonate is preferred. It is preferably chosen from sodium bicarbonate and potassium bicarbonate. Sodium bicarbonate is preferred.

The bicarbonate can be added to the aqueous composition of algae in the form of a solid, of an aqueous suspension or of an aqueous solution. Addition in the form of an aqueous solution is preferred. Without wishing to be bound by any theoretical explanation, it is thought that this type of addition has the advantage of reducing the blending time required to obtain a homogeneous distribution of the bicarbonate in the fresh algal biomass. The bicarbonate content of the aqueous solution is generally adjusted according to the dry matter content of the algal composition and to the bicarbonate content to be produced in the aqueous algal composition. The bicarbonate content in the solution, expressed as sodium bicarbonate per litre of solution, is generally at least 10 g/l, preferably at least 50 g/l and more preferably at least 80 g/l. This content is generally at most equal to the solubility of the bicarbonate at the temperature of the aqueous solution. For sodium bicarbonate, this content is generally at most 100 g/l and preferably at most 95 g/l. A content of approximately 90 g/l is particularly suitable.

In both embodiments of the invention, after addition of bicarbonate to the aqueous composition of algae, in particular when the aqueous algal composition is a fresh algal biomass and more specifically when the addition of bicarbonate is carried out via an aqueous solution of the latter, the bicarbonate-supplemented aqueous composition of algae is blended. The blending may be of manual or non-manual type, manual blending being preferred.

In both embodiments of the invention, after addition of bicarbonate to the aqueous composition of algae, in particular when the aqueous algal composition is a fresh algal biomass, the supplemented aqueous composition of algae has a storage life that is generally at least 1 day, preferably at least 2 days, more preferably at least 5 days, even more preferably at least 8 days and still more preferably at least 10 days longer than the storage life of an aqueous composition of algae that is not supplemented with bicarbonate, in particular when the aqueous algal composition is a fresh algal biomass not supplemented with bicarbonate. This storage life of the bicarbonate-supplemented aqueous composition of algae is generally greater than or equal to 4 days, preferably greater than or equal to 5 days, more particularly preferably greater than or equal to 6 days, even more particularly preferably greater than or equal to 8 days and quite particularly preferably greater than or equal to 10 days.

In the case of a bicarbonate-supplemented fresh algal biomass of spirulina, the storage life is evaluated by determining the variations in the visual appearance of the composition (decline following the breaking up of the algal cells, turning blue due to the decomposition of phycocyanin by ultraviolet radiation) and via how much release there is of nauseating sulphur-containing compounds.

The water removal step can be carried out by any operation. This operation is preferably chosen from filtering, pressing and draining operations, and combinations of at least two thereof. A filtering operation followed by a pressing and/or draining operation on the aqueous composition of algae is preferred. The water content of the aqueous composition of algae after the filtering operation is generally greater than or equal to 85% by weight of the filtered aqueous composition and less than or equal to 95% by weight. A water content of approximately 90% by weight is very suitable. The water content of the aqueous composition of algae after the filtering and pressing and/or draining operation is generally greater than or equal to 70% by weight of the filtered and pressed aqueous composition and less than 85% by weight. A water content of approximately 75% by weight is very suitable. Bicarbonate is preferably added to the aqueous composition of algae after filtering and pressing and/or draining.

In the drying process according to the invention, the bicarbonate-supplemented aqueous composition of algae is generally treated with a stream of air.

In a first aspect of the drying process, the flow rate of this stream of air is generally greater than or equal to 20 Nm³/h/kg of composition before treatment with the stream of air, and preferably greater than or equal to 35 Nm³/h/kg. This flow rate is usually less than or equal to 100 Nm³/h/kg of composition before treatment with the stream of air, and preferably less than or equal to 80 Nm³/h/kg.

In this first aspect of the drying process, the treatment with the stream of air is carried out at a temperature generally greater than or equal to 25°C, preferably greater than or equal to 30°C. This temperature is usually less than or equal to 100°C and preferably less than or equal to 80°C. A temperature of approximately 40 to 50°C is particularly suitable.

In this first aspect of the drying process, the treatment with the stream of air is carried out for a period generally greater than or equal to 1 h, preferably greater than or equal to 1.5 h. This period is usually less than or equal to 10 h and preferably less than or equal to 5 h. A period of approximately 2 to 3 h is particularly suitable.

The above conditions avoid destroying the vitamins and the essential fatty acids contained in the alga.

In this first aspect of the drying process, the latter is preferably carried out in a ventilated cabinet covered with wire netting which ensures drying away from direct light and from insects.

In this first aspect of the drying process, the aqueous composition after the addition of the bicarbonate and before the drying is preferably extruded, preferably in the form of filaments. The filaments are preferably placed on a tray covered with wire netting, preferably in a single layer, and the trays are placed in the ventilated cabinet covered with wire netting.

In this first aspect, the bicarbonate added to the aqueous composition of algae before the drying is generally not decomposed during said drying.

This first type of drying is customarily used in small-scale production of algae.

One particular mode of drying, which is used in small-scale production of algae and which is non-limiting, of the present invention is the following. A dryer, usually a ventilated solar drying oven, with racks and operating in batchwise mode is used. The algal biomass of spirulina containing approximately 75% by weight of water and supplemented with sodium bicarbonate is converted into "spaghetti"-shaped filaments by passing it through an extruder. The filaments are then spread out on racks (thin grids made from food-grade stainless steel or a polyamide sieve with a porosity of 30 to 60 µm, mounted on wooden frames). These racks are stacked on shelves placed in the dryer. This dryer makes it possible to dry approximately 12 kg of wet biomass so as to obtain, after a drying time for 2 h to 3 h and at a temperature of between 40 and 45°C, 3 kg of biomass, the moisture content of which is approximately 4% to 5% by weight. Although the drying time at low temperature is longer than at high temperature, it preserves the spirulina cells which are not subjected to direct contact with hot gases. The ventilation is not obligatory, but enables faster drying than natural convection alone.

The aqueous composition of algae, in particular the fresh algal biomass, more specifically of spirulina, treated with bicarbonate, stored for 7 days at a temperature of 1°C and dried according to the first aspect above, exhibits organoleptic qualities which are superior to the same composition not treated with bicarbonate, stored and dried under the same conditions.

The aqueous composition of algae, in particular the fresh algal biomass, more specifically of spirulina, treated with bicarbonate, stored for 7 days at a temperature of 1°C and dried according to the first aspect above, exhibits nutrient contents which are higher than the same composition not treated with bicarbonate, stored and dried under the same conditions. These nutrients are, among others, proteins, fats, vitamins A, E, K1, B1, B2, B3, B5, B6, B8 and B9 and fatty acids. The contents of these nutrients are generally analysed by the following methods:
Proteins, KJELDHAL method (N*6.25), Order of 08-09-1977 in the Journal Officiel de la République Française (JORF) [Official Journal of the French Republic];
Fats, method adapted from the order of 08/09/1977 of the JORF;
Vitamin A (Retinol), method according to French standard NF V18-401 (1997);
Vitamin E (DL-alpha-Tocopherol acetate), method according to standard NF V18-402 (1987 or 1997);
Vitamin K1 (Phylloquinone), NF EN 14148;
Vitamin B1 (Thiamine), NF EN 14122;
Vitamin B2 (Riboflavin), NF EN 14152;
Vitamin B3 or PP (Niacin or nicotinic acid), High Performance Liquid Chromatography (HPLC) method according to NF 15652;
Total vitamin B5, Ultra-Performance Liquid Chromatography tandem Mass Spectrometry (UPLC/MS/MS) method;
Vitamin B6 (Pyridoxine), NF EN 14164;
Vitamine B8 ou H (Biotin), HPLC method according to NF EN 15607;
Free vitamin B9 (folic acid) HPLC method or UPLC/MS/MS method;
Fatty acids (profile), absolute, method according to NF EN ISO 5508/12966-2.

In a second aspect of the drying process, the flow rate of this stream of air is generally greater than or equal to 800 Nm³/h/kg of composition before treatment with the stream of air, and preferably greater than or equal to 1000 Nm³/h/kg. This flow rate is usually less than or equal to 2000 Nm³/h/kg of composition before treatment with the stream of air, and preferably less than or equal to 1500 Nm³/h/kg.

In this second aspect of the drying process, the treatment with the stream of air is carried out at a temperature generally greater than or equal to 175°C, preferably greater than or equal to 185°C. This temperature is usually less than or equal to 220°C and preferably less than or equal to 210°C. A temperature of approximately 190°C to 200°C is particularly suitable.

In this second aspect of the drying process, the treatment with the stream of air is carried out for a period generally greater than or equal to 1 s, preferably greater than or equal to 2 s and more preferably greater than or equal to 5 s. This period is usually less than or equal to 30 s, preferably less than or equal to 20 s and more preferably greater than or equal to 10 s.

In this second aspect of the drying process, the latter is preferably carried out in an atomizer or in an oven.

This second type of drying is customarily used in an industrial production of algae.

The aqueous composition of algae, in particular the fresh algal biomass, more specifically of spirulina, treated with bicarbonate and dried according to the second aspect above, exhibits organoleptic qualities which are superior to the same composition not treated with bicarbonate and dried under the same conditions. These organoleptic qualities are evaluated according to the method described above.

The aqueous composition of algae, in particular the fresh algal biomass, more specifically of spirulina, treated with bicarbonate, stored for 7 days at a temperature of 1°C and dried according to the second aspect above, exhibits nutrient contents which are higher than or equal to the same composition not treated with bicarbonate, stored and dried under the same conditions. These nutrients and the methods for obtaining their contents are as described above.

The conditions described in detail above for the first and second aspects of the drying process are particularly suitable for a fresh algal biomass, preferably of spirulina, as described above for the first embodiment. The type of bicarbonate, its content and the mode of addition thereof to the fresh algal biomass are as described above for the first embodiment.

The drying process of the first aspect of the second embodiment of the invention generally comprises the following steps:
(a) Filtering the aqueous composition of algae so as to obtain a filtered aqueous composition of algae;
(b) Pressing and/or draining the filtered aqueous composition of algae of step (a) so as to obtain a pressed and/or drained aqueous composition of algae;
(c) Adding at least one bicarbonate to the pressed and/or drained aqueous composition of algae of step (b) in a total amount, relative to the amount of dry matter of the pressed and/or drained aqueous composition of algae of step (b) before addition of the bicarbonate, of at least 0.5% by weight and of at most 8% by weight;
(d) Blending the bicarbonate-supplemented, pressed and/or drained, aqueous composition of algae of step (c); and
(e) Drying the blended aqueous composition of algae of step (d) in a stream of air.

The drying process of the second aspect of the second embodiment of the invention generally comprises the following steps:
(A) Filtering the aqueous composition of algae so as to obtain a filtered aqueous composition of algae;
(B) Adding at least one bicarbonate to the filtered aqueous composition of algae of step (A) in a total amount, relative to the amount of dry matter of the pressed and/or drained aqueous composition of algae of step (A) before addition of the bicarbonate, of at least 0.5% by weight and of at most 8% by weight; and
(C) Drying the aqueous composition of algae of step (B) in a stream of air.

These processes apply particularly well to an aqueous composition of spirulina, supplemented with sodium bicarbonate.

The following example is intended to illustrate the invention without limiting the scope thereof.

### Example

An aqueous composition of spirulina from an open-air pond culture was filtered for about ten minutes on a polyamide sieve with a porosity of 50 µm. The flow of the culture medium feeder liquid through the sieve was promoted by means of a succession of spreading operations using a small plastic spade and heaping into small piles, without stressing the algae. This operation lasted approximately 10 to 15 min. At the end of this treatment, the filtered aqueous composition contained approximately 90% of water.

The filtered aqueous composition was then introduced into a small fine-mesh cloth bag and was pressed therein in a device with a simple lever arm. The pressing lasted between 30 and 45 minutes.

At the end of the pressing, the pressed aqueous composition contained approximately 75% moisture content and 25% by weight of dry matter, for a total weight of approximately 8 kg. The pressed aqueous composition was immediately stored in a refrigerator at approximately 4°C.

Two solutions of sodium bicarbonate in water were prepared by dissolving, respectively, 3.7 g and 7.6 g of bicarbonate (quality 0/13 codex) in 80 g of water.

Each of the solutions was added to 733 g of the pressed aqueous composition of algae, so as to obtain pressed and supplemented aqueous solutions of algae comprising respectively 2% and 4% by weight of sodium bicarbonate relative to the amount of dry matter of the composition before addition of the bicarbonate.

The addition of 80 g of water not containing sodium bicarbonate to 733 g of the pressed aqueous composition of algae made it possible to obtain a reference non-supplemented pressed composition of algae.

The addition operations were carried out after 3 days of storage in the refrigerator.

The paste obtained after addition was manually kneaded, slowly, for approximately 10 minutes so as to obtain pressed aqueous compositions of algae supplemented with sodium bicarbonate, and a reference composition not supplemented with bicarbonate.

Fractions of 400 g were sampled from each pressed aqueous composition of algae supplemented or not supplemented with sodium bicarbonate and were immediately stored in a leaktight box, disinfected beforehand with alcohol and then dried with compressed air, and were placed in a refrigerator at approximately 4°C.

After 6 to 7 days of storage under the latter conditions, the pressed aqueous composition of algae not supplemented with sodium bicarbonate became dark in colour with blueish glints and it gave off a nauseating odour, characteristic of a decomposition process.

After 8 days of storage under these conditions, the pressed aqueous compositions of algae supplemented at 2% and 4% with sodium bicarbonate exhibit none of the decomposition characteristics noted above.

## Claims

1. Process for drying an aqueous composition of algae, comprising one or more water removal steps, and according to which at least one bicarbonate is added to at least one of the water removal steps in a total amount, relative to the amount of dry matter of the aqueous composition of algae before addition of the bicarbonate, of at least 0.5% by weight and of at most 8% by weight, and according to which the bicarbonate-supplemented aqueous composition of algae is treated with a stream of air having a flow rate greater than or equal to 20 Nm³/h/kg of composition before treatment with the stream of air and less than or equal to 100 Nm³/h/kg, at a temperature greater than or equal to 25°C and less than or equal to 100°C and for a period greater than or equal to 1 h and less than or equal to 10 h.

2. Process according to Claim 1, in which the treatment with the stream of air is carried out at a temperature of 40°C to 50°C.

3. Process according to Claim 1 or 2, in which the treatment with the stream of air is carried out for a period of 2 to 3 h.

4. Process according to any of Claims 1 to 3, comprising the following steps:
(a) Filtering the aqueous composition of algae so as to obtain a filtered aqueous composition of algae;
(b) Pressing and/or draining the filtered aqueous composition of algae of step (a) so as to obtain a pressed and/or drained aqueous composition of algae;
(c) Adding at least one bicarbonate to the pressed and/or drained aqueous composition of algae of step (b) in a total amount, relative to the amount of dry matter of the pressed and/or drained aqueous composition of algae of step (b) before addition of the bicarbonate, of at least 0.5% by weight and of at most 8% by weight;
(d) Blending the bicarbonate-supplemented, pressed and/or drained, aqueous composition of algae of step (c); and
(e) Drying the blended aqueous composition of algae of step (d) in a stream of air.

5. Process for drying an aqueous composition of algae, comprising one or more water removal steps, and according to which at least one bicarbonate is added to at least one of the water removal steps in a total amount, relative to the amount of dry matter of the aqueous composition of algae before addition of the bicarbonate, of at least 0.5% by weight and of at most 8% by weight, and according to which the bicarbonate-supplemented aqueous composition of algae is treated with a stream of air having a flow rate greater than or equal to 800 Nm³/h/kg of composition before treatment with the stream of air and less than or equal to 2000 Nm³/h/kg, at a temperature greater than or equal to 175°C and less than or equal to 220°C and for a period greater than or equal to 1 s and less than or equal to 30 s.

6. Process according to Claim 5, in which the treatment with the stream of air is carried out at a temperature of 190°C to 200°C.

7. Process according to claim 5 or 6, in which the drying is carried out in an atomizer or in an oven.

8. Process according to any of Claims 5 to 7, comprising the following steps:
(A) Filtering the aqueous composition of algae so as to obtain a filtered aqueous composition of algae;
(B) Adding at least one bicarbonate to the filtered aqueous composition of algae of step (A) in a total amount, relative to the amount of dry matter of the pressed and/or drained aqueous composition of algae of step (A) before addition of the bicarbonate, of at least 0.5% by weight and of at most 8% by weight; and
(C) Drying the aqueous composition of algae of step (B) in a stream of air.

9. Process according to any of the preceding Claims, in which the aqueous composition of algae contains, before addition of the bicarbonate, a total amount of water of at least 60% by weight and at most 90% by weight of the composition.

10. Process according to any of the preceding Claims, in which the aqueous composition of algae contains, before addition of the bicarbonate, a total amount of water of at least 70% by weight and at most 80 % by weight of the composition.

11. Process according to any of the preceding Claims, in which the aqueous composition of algae is a fresh algal biomass, preferably a fresh biomass of spirulina.

12. Process according to any of the preceding Claims, in which the bicarbonate is chosen from alkali metal bicarbonates and ammonium bicarbonate, preferably from alkali metal bicarbonates and particularly preferably sodium bicarbonate.

13. Process according to any of the preceding Claims, in which the bicarbonate is added to the aqueous composition of algae in the form of an aqueous solution.

14. Process according to Claim 13, in which the bicarbonate content in the aqueous solution is at least 10 g/l, preferably at least 50 g/l and most preferably at least 80 g/l.

15. Process according to Claims 4 or 8, in which the water content of the aqueous composition of algae after the filtering operation is greater than or equal to 85% by weight of the filtered aqueous composition.

## Patentansprüche

1. Verfahren zum Trocknen einer wässrigen Algenzusammensetzung, umfassend einen oder mehrere Wasserentfernungsschritte, und wobei demgemäß mindestens ein Bicarbonat mindestens einem der Wasserentfernungsschritte in einer Gesamtmenge, bezogen auf die Menge an Trockenmaterial der wässrigen Algenzusammensetzung vor der Zugabe des Bicarbonats, von mindestens 0,5 Gew.% und höchstens 8 Gew.% zugegeben wird, und demgemäß die mit Bicarbonat ergänzte wässrige Algenzusammensetzung mit einem Luftstrom mit einer Flussrate größer als oder gleich 20 Nm³/h/kg der Zusammensetzung vor der Behandlung mit dem Luftstrom und weniger als oder gleich 100 Nm³/h/kg bei einer Temperatur größer als oder gleich 25 °C und kleiner als oder gleich 100 °C und für einen Zeitraum größer als oder gleich 1 h und kleiner als oder gleich 10 h behandelt wird.

2. Verfahren nach Anspruch 1, wobei die Behandlung mit dem Luftstrom bei einer Temperatur von 40 °C bis 50 °C durchgeführt wird.

3. Verfahren nach Anspruch 1 oder 2, wobei die Behandlung mit dem Luftstrom für einen Zeitraum von 2 bis 3 h durchgeführt wird.

4. Verfahren nach einem der Ansprüche 1 bis 3, welches die folgenden Schritte umfasst:
(a) Filtrieren der wässrigen Algenzusammensetzung, um so eine filtrierte wässrige Algenzusammensetzung zu erhalten;
(b) Pressen und/oder Drainieren der filtrierten wässrigen Algenzusammensetzung aus Schritt (a), um so eine gepresste und/oder drainierte wässrige Algenzusammensetzung zu erhalten;
(c) Zugeben von mindestens einem Bicarbonat zu der gepressten und/oder drainierten wässrigen Algenzusammensetzung aus Schritt (b) in einer Gesamtmenge, bezogen auf die Menge des Trockenmaterials der gepressten und/oder drainierten wässrigen Algenzusammensetzung aus Schritt (b), vor der Zugabe des Bicarbonats, von mindestens 0,5 Gew.% und höchstens 8 Gew.%;
(d) Vermischen der mit Bicarbonat ergänzten, gepressten und/oder drainierten wässrigen Algenzusammensetzung aus Schritt (c) und
(e) Trocknen der vermischten wässrigen Algenzusammensetzung aus Schritt (d) in einem Luftstrom.

5. Verfahren zum Trocknen einer wässrigen Algenzusammensetzung, umfassend einen oder mehrere Wasserentfernungsschritte, und wobei demgemäß mindestens ein Bicarbonat mindestens einem der Wasserentfernungsschritte in einer Gesamtmenge, bezogen auf die Menge an Trockenmaterial der wässrigen Algenzusammensetzung vor der Zugabe des Bicarbonats, von mindestens 0,5 Gew.% und höchstens 8 Gew.% zugegeben wird, und demgemäß die mit Bicarbonat ergänzte wässrige Algenzusammensetzung mit einem Luftstrom mit einer Flussrate größer als oder gleich 800 Nm³/h/kg der Zusammensetzung vor der Behandlung mit dem Luftstrom und weniger als oder gleich 2000 Nm³/h/kg bei einer Temperatur größer als oder gleich 175 °C und kleiner als oder gleich 220 °C und für einen Zeitraum größer als oder gleich 1 s und kleiner als oder gleich 30 s behandelt wird.

6. Verfahren nach Anspruch 5, wobei die Behandlung mit dem Luftstrom bei einer Temperatur von 190 °C bis 200 °C durchgeführt wird.

7. Verfahren nach Anspruch 5 oder 6, wobei das Trocknen in einem Zerstäuber oder einem Ofen durchgeführt wird.

8. Verfahren nach einem der Ansprüche 5 bis 7, welches die folgenden Schritte umfasst:
(A) Filtrieren der wässrigen Algenzusammensetzung, um so eine filtrierte wässrige Algenzusammensetzung zu erhalten;
(B) Zugeben von mindestens einem Bicarbonat zu der filtrierten wässrigen Algenzusammensetzung aus Schritt (A) in einer Gesamtmenge, bezogen auf die Menge des Trockenmaterials der gepressten und/oder drainierten wässrigen Algenzusammensetzung aus Schritt (A) vor der Zugabe des Bicarbonats, von mindestens 0,5 Gew.% und höchstens 8 Gew.%; und
(C) Trocknen der wässrigen Algenzusammensetzung aus Schritt (B) in einem Luftstrom.

9. Verfahren nach einem der vorhergehenden Ansprüche, wobei die wässrige Algenzusammensetzung vor der Zugabe des Bicarbonats eine Gesamtmenge an Wasser von mindestens 60 Gew.% und höchstens 90 Gew.% der Zusammensetzung enthält.

10. Verfahren nach einem der vorhergehenden Ansprüche, wobei die wässrige Algenzusammensetzung vor der Zugabe des Bicarbonats eine Gesamtmenge an Wasser von mindestens 70 Gew.% und höchstens 80 Gew.% der Zusammensetzung enthält.

11. Verfahren nach einem der vorhergehenden Ansprüche, wobei die wässrige Algenzusammensetzung eine Frischalgenbiomasse ist, vorzugsweise eine Frischbiomasse von Spirulina.

12. Verfahren nach einem der vorhergehenden Ansprüche, wobei das Bicarbonat ausgewählt ist aus Alkalimetallbicarbonaten und Ammoniumbicarbonat, vorzugsweise aus Alkalimetallbicarbonaten und besonders bevorzugt Natriumbicarbonat.

13. Verfahren nach einem der vorhergehenden Ansprüche, wobei das Bicarbonat der wässrigen Algenzusammensetzung in Form einer wässrigen Lösung zugegeben wird.

14. Verfahren nach Anspruch 13, wobei der Bicarbonatgehalt in der wässrigen Lösung mindestens 10 g/l, vorzugsweise mindestens 50 g/l und am meisten bevorzugt mindestens 80 g/l beträgt.

15. Verfahren nach Anspruch 4 oder 8, wobei der Wassergehalt der wässrigen Algenzusammensetzung nach der Filtration größer als oder gleich 85 Gew.% der filtrierten wässrigen Zusammensetzung ist.

## Revendications

1. Procédé de séchage d'une composition aqueuse d'algues, comprenant une ou plusieurs étapes d'élimination d'eau, et selon lequel au moins un bicarbonate est ajouté à au moins l'une des étapes d'élimination d'eau selon une quantité totale, par rapport à la quantité de matière sèche de la composition aqueuse d'algues avant l'addition du bicarbonate, d'au moins 0,5% en poids et d'au plus 8% en poids, et selon lequel la composition aqueuse d'algues complémentée en bicarbonate est traitée par un courant d'air ayant un débit supérieur ou égal à 20 Nm³/h/kg de composition avant traitement par le courant d'air et inférieur ou égal à 100 Nm³/h/kg, à une température supérieure ou égale à 25°C et inférieure ou égale à 100°C et pendant une période supérieure ou égale à 1 h et inférieure ou égale à 10 h.

2. Procédé selon la revendication 1, dans lequel le traitement par le courant d'air est effectué à une température allant de 40°C à 50°C.

3. Procédé selon la revendication 1 ou 2, dans lequel le traitement par le courant d'air est effectué pendant une période allant de 2 à 3 h.

4. Procédé selon l'une quelconque des revendications 1 à 3, comprenant les étapes suivantes :
(a) la filtration de la composition aqueuse d'algues de façon à obtenir une composition aqueuse d'algues filtrée ;
(b) le pressage et/ou le drainage de la composition aqueuse d'algues filtrée de l'étape (a) de façon à obtenir une composition aqueuse d'algues pressée et/ou drainée ;
(c) l'addition d'au moins un bicarbonate à la composition aqueuse d'algues pressée et/ou drainée de l'étape (b) selon une quantité totale, par rapport à la quantité de matière sèche de la composition aqueuse d'algues pressée et/ou drainée de l'étape (b) avant l'addition du bicarbonate, d'au moins 0,5% en poids et d'au plus 8% en poids ;
(d) le mélange de la composition aqueuse d'algues pressée et/ou drainée, complémentée par du bicarbonate, de l'étape (c) ; et
(e) le séchage de la composition aqueuse d'algues mélangée de l'étape (d) dans un courant d'air.

5. Procédé de séchage d'une composition aqueuse d'algues, comprenant une ou plusieurs étapes d'élimination d'eau, et selon lequel au moins un bicarbonate est ajouté à au moins l'une des étapes d'élimination d'eau selon une quantité totale, par rapport à la quantité de matière sèche de la composition aqueuse d'algues avant l'addition du bicarbonate, d'au moins 0,5% en poids et d'au plus 8% en poids, et selon lequel la composition aqueuse d'algues complémentée en bicarbonate est traitée par un courant d'air ayant un débit supérieur ou égal à 800 Nm³/h/kg de composition avant traitement par le courant d'air et inférieur ou égal à 2000 Nm³/h/kg, à une température supérieure ou égale à 175°C et inférieure ou égale à 220°C et pendant une période supérieure ou égale à 1 s et inférieure ou égale à 30 s.

6. Procédé selon la revendication 5, dans lequel le traitement par le courant d'air est effectué à une température allant de 190°C à 200°C.

7. Procédé selon la revendication 5 ou 6, dans lequel le séchage est effectué dans un atomiseur ou dans un four.

8. Procédé selon l'une quelconque des revendications 5 à 7, comprenant les étapes suivantes :
(a) la filtration de la composition aqueuse d'algues de façon à obtenir une composition aqueuse d'algues filtrée ;
(b) l'addition d'au moins un bicarbonate à la composition aqueuse d'algues filtrée de l'étape (a) selon une quantité totale, par rapport à la quantité de matière sèche de la composition aqueuse d'algues pressée et/ou drainée de l'étape (a) avant l'addition du bicarbonate, d'au moins 0,5% en poids et d'au plus 8% en poids ; et
(c) le séchage de la composition aqueuse d'algues de l'étape (b) dans un courant d'air.

9. Procédé selon l'une quelconque des revendications précédentes, dans lequel la composition aqueuse d'algues contient, avant l'addition du bicarbonate, une quantité totale d'eau d'au moins 60% en poids et d'au plus 90% en poids de la composition.

10. Procédé selon l'une quelconque des revendications précédentes, dans lequel la composition aqueuse d'algues contient, avant l'addition du bicarbonate, une quantité totale d'eau d'au moins 70% en poids et d'au plus 80% en poids de la composition.

11. Procédé selon l'une quelconque des revendications précédentes, dans lequel la composition aqueuse d'algues est une biomasse algale fraîche, préférablement une biomasse fraîche de spiruline.

12. Procédé selon l'une quelconque des revendications précédentes, dans lequel le bicarbonate est choisi parmi les bicarbonates de métaux alcalins et le bicarbonate d'ammonium, préférablement parmi les bicarbonates de métaux alcalins et particulièrement préférablement le bicarbonate de sodium.

13. Procédé selon l'une quelconque des revendications précédentes, dans lequel le bicarbonate est ajouté à la composition aqueuse d'algues sous la forme d'une solution aqueuse.

14. Procédé selon la revendication 13, dans lequel la teneur en bicarbonate dans la solution aqueuse est d'au moins 10 g/l, préférablement d'au moins 50 g/l et tout préférablement d'au moins 80 g/l.

15. Procédé selon les revendications 4 ou 8, dans lequel la teneur en eau de la composition aqueuse d'algues après l'opération de filtration est supérieure ou égale à 85% en poids de la composition aqueuse filtrée.
